# EUROPEAN PATENT SPECIFICATION

(11) **EP 3 193 911 B1**
(45) Date of publication and mention of the grant of the patent: **06.09.2023**
(21) Application number: 15741222.2
(22) Date of filing: 24.07.2015
(51) Int. Cl.: A61K 38/37, A61P 7/04

(54) **IMPROVED FACTOR VIII PREPARATIONS FOR USE IN TREATING HEMOPHILIA A**
VERBESSERTE FAKTOR-VIII-PRÄPARATE ZUR VERWENDUNG IN DER BEHANDLUNG VON HÄMOPHILIE A
PRÉPARATIONS DE FACTEUR VIII AMÉLIORÉES POUR L'UTILISATION DANS LE TRAITEMENT DE L'HÉMOPHILIE A

(30) Priority: 25.07.2014 EP 14178563
(43) Date of publication of application: 26.07.2017
(73) Proprietor: CSL Behring GmbH, 35041 Marburg (DE)
(72) Inventor: SCHMIDBAUER, Stefan, 35094 Lahntal (DE); METZNER, Hubert, 35041 Marburg (DE); ROBBEL, Lars, 35083 Wetter (DE)
(74) Representative: CSL Global IP
(86) International application number: PCT/EP2015/067014
(87) International publication number: WO 2016/012595

(56) References cited:
- WO-A1-00/23116
- WO-A1-91/09122
- WO-A1-2013/057219
- WAKABAYASHI HIRONAO ET AL: "Ca2+ binding to both the heavy and light chains of factor VIII is required for cofactor activity", BIOCHEMISTRY, AMERICAN CHEMICAL SOCIETY, US, vol. 41, no. 26, 2 July 2002 (2002-07-02), pages 8485-8492, XP002410844, ISSN: 0006-2960, DOI: 10.1021/BI025589O

## Description

### Field of the invention:

The present invention relates to pharmaceutical preparations comprising two-chain Factor VIII that are essentially free of Factor VIII-Light Chains (FVIII-LCs) which are not associated with Factor VIII-Heavy Chains (FVIII-HCs) having higher purity and which are less immunogenic.

### Background of the invention:

There are various bleeding disorders caused by deficiencies of blood coagulation factors. Some of the most common bleeding disorders are hemophilia A and B, resulting from deficiencies of blood coagulation Factor VIII and IX, respectively.

Classic hemophilia or hemophilia A is an inherited bleeding disorder. It results from a chromosome X-linked deficiency of blood coagulation Factor VIII, and affects almost exclusively males with an incidence of between one and two individuals per 10.000. The X-chromosome defect is transmitted by female carriers who are not themselves hemophiliacs. The clinical manifestation of hemophilia A is an increased bleeding tendency. Before treatment with Factor VIII concentrates was introduced the mean life span for a person with severe hemophilia was less than 20 years. The use of concentrates of Factor VIII from plasma has considerably improved the situation for the hemophilia A patients increasing the mean life span extensively, giving most of them the possibility to live a more or less normal life. However, there have been certain problems with the plasma derived concentrates and their use, the most serious of which have been the transmission of viruses. So far, viruses causing hepatitis B, non-A non-B hepatitis and AIDS have hit the population seriously. Since then different virus inactivation methods and new highly purified Factor VIII concentrates have been developed which established a very high safety standard also for plasma derived Factor VIII.

The cloning of the cDNA for Factor VIII (Wood et al. 1984. Nature 312:330-336; Vehar et al. 1984. Nature 312:337-342) made it possible to express Factor VIII recombinantly leading to the development of several recombinant Factor VIII products, which were approved by the regulatory authorities between 1992 and 2003. The fact that the central B domain of the Factor VIII polypeptide chain residing between amino acids Arg-740 and Glu-1649 does not seem to be necessary for full biological activity has also led to the development of B domain deleted Factor VIII products.

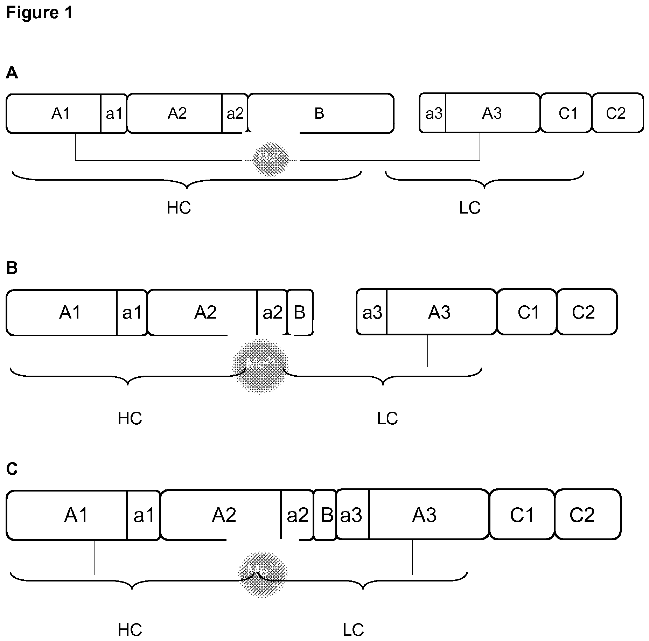

The mature wild-type Factor VIII molecule consists of 2332 amino acids which can be grouped into three homologous A domains, two homologous C domains and a B Domain which are arranged in the order: A1-A2-B-A3-C1-C2. The complete amino acid sequence of mature human Factor VIII is shown in SEQ ID NO:2. The A domains also comprise acidic regions highlighted as a1, a2, and a3 (Figure 1). During its secretion into plasma Factor VIII is processed intracellularly into a series of heterodimers of different length which are associated by metal-ions as single chain Factor VIII is cleaved at the B-A3 boundary and at different sites within the B-domain. This processing leads to heterogeneous heavy chain molecules consisting of the A1, the A2 and various parts of the B-domain which have a molecular size ranging from 90 kDa to 200 kDa and to light chain molecules which have a molecular size of about 80 kDa. One heavy chain is bound non-covalently via a metal ion bridge to one light chain, which consist of the A3, the C1 and the C2 domain (Saenko et al. 2002. Vox Sang. 83:89-96). In plasma this heterodimeric Factor VIII binds with high affinity to von Willebrand Factor, which protects it from premature catabolism. The half-life of non-activated Factor VIII bound to vWF is about 12 hours in plasma.

Coagulation Factor VIII is activated via proteolytic cleavage by FXa and thrombin at amino acids Arg372 and Arg740 within the heavy chain and at Arg1689 within the light chain resulting in the release of von Willebrand Factor and generating the activated Factor VIII heterotrimer which will form the tenase complex on phospholipid surfaces with FIXa and FX provided that Ca²⁺ is present. The heterotrimer consists of the A1 domain, a 50 kDa fragment, the A2 domain, a 43 kDa fragment and the light chain (A3-C1-C2), which is a fragment of about 80 kDa. Thus the active form of Factor VIII (Factor Villa) consists of an A1-subunit associated through the divalent metal ion linkage to a thrombin-cleaved A3-C1-C2 light chain and a free A2 subunit relatively loosely associated with the A1 and the A3 domain.

Due to the introduction of virus inactivation and elimination steps in the production processes of plasma derived and recombinant Factor VIII concentrates the virus safety of these products is very high. Therefore, the most serious side effect of Factor VIII treatment nowadays is the development of inhibitory antibodies to Factor VIII. This may result in the neutralization of the administered Factor VIII concentrate leading sometimes to life-threatening bleeding episodes. It has been described that in the treatment of hemophilia A the risk of the patient to develop an inhibitor throughout its life is in the range of about 25-30%.

There is, therefore, an unmatched clinical need to provide Factor VIII products, i.e. recombinant Factor VIII products with a reduced immunogenicity.

It has been described, that the risk of inhibitor generation in previously untreated patients with Haemophilia A is higher when treated with recombinant Factor VIII products, and it has been speculated that the binding of vWF to different epitopes in the FVIII-LC (A3 and C2 domains) shields these epitopes and might therefore have an beneficial effect in reducing immunogenicity (C. Escuriola Ettinghausen, W. Kreuz; Haemophilia (2006), 12, (Suppl. 6), 102-106). Unshielded epitopes of Factor VIII appears to pose a risk of triggering the generation of inhibitors. In addition, the interaction of the FVIII-HC and the FVIII-LC in the two-chain FVIII complex is assumed to also have a shielding effect on otherwise freely accessible epitopes in the FVIII-HC and the FVIII-LC.

The isolation of Factor VIII from human plasma (pdFVIII) or the recombinant production of Factor VIII (rFVIII) nowadays are well-known procedures to provide Haemophilia A patients with the corresponding concentrates for treatment. However, during manufacturing these Factor VIII products are subjected to unphysiologic conditions which may result in damage and partial inactivation. This may be caused by proteolytic inactivation or by chemical or structural modification. Such structural modifications as e.g. the disruption of the interaction of the different domains may be triggered by loss of stabilizing metal ions or by contact to foreign surfaces. In WO 97/33178 it has been described that proteolytic Factor VIII fragments in a molecular weight range between 20 and 50 kDa in a pharmaceutical Factor VIII preparation may be critical for giving rise to inhibitor formation and a process was described using size exclusion chromatography (SEC) where Factor VIII fractions containing such fragments are separated to obtain a Factor VIII product with a lower content of these fragments and thus lower immunogenicity. However, WO 97/33178 did not mention the separation of the Factor VIII preparation from Factor VIII fragments larger than 50 kDa. US 4,675,385, EP0321835, EP0399321 and EP0410207 describe the use of size exclusion chromatography for the preparation of Factor VIII but none of these patents describes the presence nor the specific removal of non-associated Factor VIII heavy chains (FVIII-HCs) or of non-associated Factor VIII light chains (Factor VIII-LCs). Native SEC was also used to analyze various commercially available Factor VIII preparations (Jankowski et al., Haemophilia (2007), 13, 30-37) but it was not recognized that non-associated FVIII-HCs or non-associated FVIII-LCs were present in these preparations.

It has now surprisingly been found that all tested commercially obtainable pharmaceutical two-chain Factor VIII preparations do comprise some amount of non-associated FVIII-LCs. As in intact Factor VIII molecules the FVIII-HCs are associated with FVIII-LCs in a metal-dependent association these non-associated FVIII-LCs do expose epitopes which are normally shielded in the metal-dependent association with FVIII-HCs and their presence in a pharmaceutical Factor VIII preparation does thus increase the risk that such Factor FVIII preparations are immunogenic. Probably in these tc-FVIII preparations also more or less FVIII-HCs which are non-associated with FVIII-LC are present and do then also pose a risk of being immunogenic. In pharmaceutical single-chain Factor VIII preparations as described in WO 2004/067566 such non-associated FVIII-HCs and FVIII-LCs will be present - if at all - in a much lower amount as there is a covalent linkage between the FVIII-HC and the FVIII-LC. However also in single-chain Factor VIII preparations there is a minority of molecules which are cleaved between the FVIII-HC and the FVIII-LC and thus this minority of tc-FVIII molecules may become partly separated into non-associated FVIII-HCs and non-associated FVIII-LCs. The invention is thus directed to pharmaceutical two-chain Factor VIII preparations with low amounts of non-associated FVIII-HCs and/or low amounts of non-associated-FVIII-LCs.

### Brief Description of the Drawings

Figure 1: Structure of full-length two-chain Factor VIII (A), B-domain-truncated two-chain Factor VIII (B) and B-domain-truncated single-chain Factor VIII (C) molecules
   (A) Two-chain full-length Factor VIII where the FVIII-HC and the FVIII-LC are associated non-covalently by a metal dependent association.
   (B) Two-chain B-domain truncated Factor VIII where the FVIII-HC and the FVIII-LC are associated non-covalently by a metal dependent association.
   (C) Single-chain B-domain truncated Factor VIII where the FVIII-HC and the FVIII-LC are associated covalently, e.g. by deletion of the furin cleavage site.
Figure 2: Representative SE-HPLC profile obtained for full-length rFVIII separated on a Yarra SEC-3000@ column under isocratic conditions. The collected fraction is indicated in the chromatogram.
Figure 3: Representative SE-HPLC profile obtained for full-length rFVIII separated on a Yarra SEC-3000@ column under isocratic conditions. The three collected fractions are indicated in the chromatogram.
Figure 4: Analytical SE-HPLC profiles obtained for the collected fractions 1 to 3 of full-length rFVIII separated on a Yarra SEC-3000@ column under isocratic conditions
Figure 5: SE-HPLC profile obtained for BDD rFVIII (ReFacto AF@) separated on a Yarra SEC-3000@ column under isocratic conditions. The five collected fractions are indicated in the chromatogram.
Figure 6: Analytical SE-HPLC profiles obtained for the collected fractions of BDD rFVIII separated on a Yarra SEC-3000@ column under isocratic conditions.
Figure 7 (A to D): Analytical SE-HPLC profiles obtained for the relevant pools/fractions of BDD rFVIII analyzed on a COSMOSIL^{®} SEC-column under isocratic conditions (chromatograms were normalized).

### Brief Summary of the Invention

The invention is as defined in claims 1 to 11.
and is directed to pharmaceutical preparations comprising two-chain Factor VIII (tc-FVIII) that are essentially free of Factor VIII Light Chains (FVIII-LCs) which are non-associated with Factor VIII Heavy Chains (FVIII-HCs)awherein less than 0.9% of all FVIII-LCs within said preparation are non-associated with FVIII-HCs.

In one embodiment of the invention the tc-FVIII is a full-length Factor VIII, which may be of plasmatic or of recombinant origin.

In specific embodiments of full-length FVIII molecules the molecular weight of the non-associated FVIII-LCs is about 70 to 125 kDa, preferably about 80 kDa.

In another embodiment the tc-FVIII is a B-domain truncated Factor VIII.

Other embodiments of the invention are directed to pharmaceutical preparations of tc-FVIII wherein the pharmaceutical preparations are less immunogenic as compared to pharmaceutical preparations comprising tc-FVIII wherein 0.9% or more of all FVIII-LCs within said preparation are non-associated with FVIII-HCs.

Certain embodiments of the invention relate to the use of the above preparations in medicine, preferably to the use in the treatment of hemophilia A.

### Description of the invention

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e., to at least one) of the grammatical object of the article. By way of example, "an element" means one element or more than one element.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight, length, or other unit described herein.

In the specification, the singular forms also include the plural and vice versa unless the context clearly dictates otherwise.

Throughout this specification, unless the context requires otherwise, the words "comprise," "comprises," and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

### Factor VIII

The terms "blood coagulation Factor VIII", "Factor VIII" and FVIII" are used interchangeably herein. "Blood coagulation Factor VIII" includes wild type blood coagulation Factor VIII as well as derivatives of wild type blood coagulation Factor VIII having the procoagulant activity of wild type blood coagulation Factor VIII. Derivatives may have deletions, insertions and/or additions compared with the amino acid sequence of wild type Factor VIII. The term Factor VIII includes proteolytically processed forms of Factor VIII, e.g. the form before activation, comprising heavy chain and light chain.

The term "Factor VIII" includes any Factor VIII variants or mutants having at least 10%, preferably at least 25%, more preferably at least 50%, most preferably at least 75% of the specific molar biological activity of wild type Factor VIII. The biological activity of Factor VIII can be determined with assays known to the expert that for example are one-stage (OS) Factor VIII clotting assays using Factor VIII-depleted plasma and an activator of the intrinsic coagulation system as reagents or chromogenic substrate (CS) Factor VIII activity assays using commercially available corresponding assay kits.

The term "Factor VIII heavy chain" or "Factor VIII HC" or "FVIII-HC" relates to a subunit of Factor VIII composed of part of the mature protein that covers in terms of the wild-type Factor VIII molecule amino acids 1 to 740 (i.e. the A1 and the A2 domain) and larger if containing still parts of the Factor VIII B domain. The FVIII-HCs of full-length FVIII do have a molecular weight of between 80 and 200 kDa depending on how much of the B-domain is retained. The molecular weight of a FVIII-HC of a B-domain truncated FVIII is at least 80 kDa and depending on how much of the original B-domain is still retained may be up to 85 kDa, or at least 90 kDa or at least 95 kDa or up to 100 kDa or up to 105 kDa or up to 110 kDa or up to 120 kDa or up to 130 kDa or up to 140 kDa or up to 150 kDa or up to 160 kDa or up to 170 kDa or up to 180 kDa or up to 190 kDa or up to 200 kDa.

The term "Factor VIII light chain" or "Factor VIII LC" or "FVIII-LC" relates to a subunit of Factor VIII composed of part of the mature protein that covers in terms of the wild-type Factor VIII molecule amino acids 1649 to 2332 (i.e. the A3, the C1 and the C2 domain). The FVIII-LCs of full-length FVIII do have usually a molecular weight of about 80 kDa but can be found between 70 kDa and 125 kDa. Jankowski et al. published (Jankowski et al., Haemophilia (2007), 13, 30-37) that the Factor VIII preparation sold under the tradename Advate^{®} comprises in addition to the normal 80 kDa FVIII-LC (cleaved before Glu1649) also a species cleaved before Asp1658 and one with a cleavage before Ala1314, the latter leading to a FVIII-LC of a molecular weight of about 125 kDa. The FVIII-LCs of B-domain truncated FVIII and of full length FVIII are of comparable length.

Factor VIII, tc-FVIII, FVIII-HC and FVIII-LC or FVIII-HCs and FVIII-LCs comprise also variants like deletions, insertions, point mutations of the wild-type Factor VIII sequences. Included are also modified Factor or Factor VIII fragments like fusions to any polypeptides like albumin, Fc parts of immunoglobulins, CTP or XTEN. Also encompassed are FVIII-HCs and FVIII-LCs which are covalently linked to any half-life extending moieties like PEG, HES or polysialic acid. Whenever molecular weights relating to Factor VIII are mentioned within this specification, they relate to the wild-type Factor VIII and any the molecular weight of any modification like a fusion protein or a PEG moiety is not comprised in this molecular weight.

In wild-type Factor VIII, i.e. in all Factor VIII preparations of plasmatic origin and also in all currently obtainable Factor VIII preparations of recombinant origin Factor VIII is predominantly present as a two-chain hetero-dimer consisting of one FVIII-HC and one FVIII-LC since the nascent Factor VIII is cleaved intracellularly within the Golgi apparatus between Arg1648 and Glu1649 and in various parts of the B-domain (if present). The two chains are held together in a metal dependent association, which appears to be mainly due to a copper ion which occupies as cupric ion (Cu⁺) a site in the A1 domain comprising His267, Cys310 and His315. The role of a second copper ion bound in the A3 domain by His1954, Cys2000 and His2005 and of a calcium bound within the A1 domain by Glu110, Asp116, Asp125, Asp126, Glu122 and Lys107 regarding the association of the two Factor VIII chains is less clear. Also a Zn²⁺ binding site within the A3 domain has been described.

The term "two-chain Factor VIII" or "tc-FVIII" as used herein relates to a Factor VIII which is cleaved during the secretion process within the tc-FVIII expressing cell intracellularly or has been reconstituted from independently expressed FVIII-HC and FVIII-LC such that it consists of two polypeptidic chains: the FVIII-HC (comprising the A1, the A2 domain) and optionally various lengths of the B-domain is associated with the FVIII-LC (comprising the A3 domain, the C1 and the C2 domain) in a metal-dependent association. Therefore in a tc-FVIII the FVIII-HC is not connected to the FVIII-LC by a peptidic linkage.

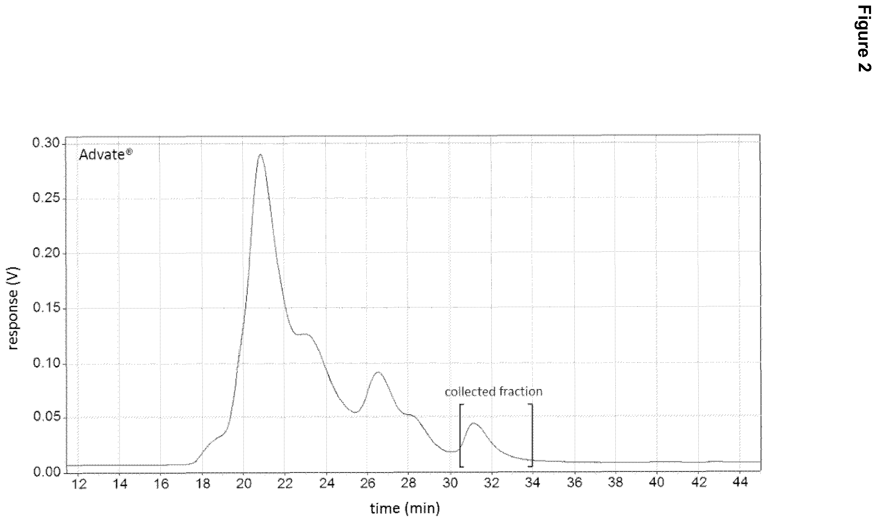

The term "associated FVIII-HC" or "associated FVIII-LC" means a FVIII-HC being part of a stable heterodimer with a FVIII-LC or a FVIII-LC being part of a stable heterodimer with a FVIII-HC, which heterodimers will elute as a heterodimer in a native size-exclusion chromatography as described by Jankowski et al. (Figure 2a, Jankowski et al., Haemophilia (2007), 13, 30-37). This association of a FVIII-HC to a FVIII-LC is mainly due to the metal dependent association as described above. As depicted in Figure 1 (A) and (B) one molecule FVIII-HC of a two-chain FVIII is associated in a metal dependent association to one molecule of FVIII-LC or vice versa.

The term "non-associated FVIII-HC" or "non-associated FVIII-LC" means a FVIII-HC not part of a stable heterodimer with a FVIII-LC or a FVIII-LC which is not part of a stable heterodimer with a FVIII-HC and which non-associated FVIII-HCs and/or non-associated FVIII-LCs will therefore not elute as part of a heterodimer in a native size-exclusion chromatography as described by Jankowski et al. (Figure 2a, Jankowski et al., Haemophilia (2007), 13, 30-37) but rather as monomers of non-associated FVIII-HCs and non-associated FVIII-LCs.

As non-limiting examples, Factor VIII molecules, or FVIII-HCs or FVIII-LCs derived therefrom include Factor VIII mutants preventing or reducing APC cleavage (Amano 1998. Thromb. Haemost. 79:557-563), Factor VIII mutants further stabilizing the A2 domain (WO 97/40145), Factor VIII mutants resulting in increased expression (Swaroop et al. 1997. JBC 272:24121-24124), Factor VIII mutants reducing its immunogenicity by exchange of amino acid sequences (Lollar 1999. Thromb. Haemost. 82:505-508), Factor VIII reconstituted from differently expressed heavy and light chains (Oh et al. 1999. Exp. Mol. Med. 31:95-100), Factor VIII mutants reducing binding to receptors leading to catabolism of Factor VIII like HSPG (heparan sulfate proteoglycans) and/or LRP (low density lipoprotein receptor related protein) (Ananyeva et al. 2001. TCM, 11:251-257), disulfide bond-stabilized Factor VIII variants (Gale et al., 2006. J. Thromb. Hemost. 4:1315-1322), Factor VIII mutants with improved secretion properties (Miao et al., 2004. Blood 103:3412-3419), Factor VIII mutants with increased cofactor specific activity (Wakabayashi et al., 2005. Biochemistry 44:10298-304), Factor VIII mutants with improved biosynthesis and secretion, reduced ER chaperone interaction, improved ER-Golgi transport, increased activation or resistance to inactivation and improved half-life (summarized by Pipe 2004. Sem. Thromb. Hemost. 30:227-237). Also pharmaceutical preparations based on porcine Factor VIII have been used to treat patients. All of these Factor VIII mutants and variants are incorporated herein by reference in their entirety.

A suitable test to determine the biological activity of Factor VIII is the one stage or the two stage coagulation assay (Rizza et al. 1982. Coagulation assay of Factor VIII:C and FIXa in Bloom ed. The Hemophilias. NY Churchchill Livingston 1992) or the chromogenic substrate Factor VIII:C assay (S. Rosen, 1984. Scand J Haematol 33: 139-145, suppl.). The content of these references is incorporated herein by reference.

Surprisingly it has now been found using native analytical SE-HPLC that all tested commercial tc-FVIII preparations contain between about 0.9% (Refacto^{®}) and about 5% (Advate^{®}) of non-associated FVIII-LCs. Non-associated FVIII-HCs may also be present in these preparations. Therefore pharmaceutical preparations of Refacto^{®} represent the closest prior art.

Factor VIII preparations of the invention comprise less than 0.9% non-associated FVIII-LCs, or less than 0.8% non-associated FVIII-LCs, or less than 0.7% non-associated FVIII-LCs, or less than 0.6% non-associated FVIII-LCs, or less than 0.5% non-associated FVIII-LCs, or less than 0.4% non-associated FVIII-LCs, or less than 0.3% non-associated FVIII-LCs, or less than 0.2% non-associated FVIII-LCs, or less than 0.1% non-associated FVIII-LCs. Most preferably tc-FVIII preparations of the invention are essentially free of non-associated FVIII-LCs.

Less than x% non-associated FVIII-LCs means, that less than x% of FVIII-LCs of all FVIII-LCs in a given Factor preparation are non-associated.

The Factor VIII in the Factor VIII preparations of the invention is preferably human Factor VIII and can be full-length Factor VIII of either plasmatic or of recombinant origin or recombinant B-domain truncated Factor VIII. "Of plasmatic origin" means purified from plasma, preferably from human plasma. "Of recombinant origin" means expressed by host cells which have been modified by recombinant technology to express Factor VIII. "B-domain truncated Factor VIII" means a Factor VIII which is encoded by a cDNA which has been modified such that it does not code for a Factor VIII with a complete wild-type B-domain, but only smaller fragment of the B-domain or no B-domain at all.

The cDNA sequence and the amino acid sequence of the mature wild type form of human blood coagulation Factor VIII are shown in SEQ ID NO:1 and SEQ ID NO:2, respectively. The reference to an amino acid position of a specific sequence means the position of said amino acid in the Factor VIII wild-type protein and does not exclude the presence of mutations, e.g. deletions, insertions and/or substitutions at other positions in the sequence referred to. For example, a mutation in "Glu2004" referring to SEQ ID NO:2 does not exclude that in the modified homologue one or more amino acids at positions 1 through 2332 of SEQ ID NO:2 are missing.

The recombinant Factor VIII protein, which accumulates in the medium of secreting cells of the above types, can be concentrated and purified by a variety of biochemical and chromatographic methods, including methods utilizing differences in size, charge, hydrophobicity, solubility, specific affinity.

An example of such purification is the adsorption of the recombinant mutant protein to a monoclonal antibody or antibody fragment which is immobilised on a solid support. After adsorption of the Factor VIII mutant to the support, washing and desorption, the protein can be further purified by a variety of chromatographic techniques based on the above properties. The order of the purification steps is chosen e.g. according to capacity and selectivity of the steps, stability of the support or other aspects. Preferred purification steps for example comprise but are not limited to ion exchange chromatography steps, immune affinity chromatography steps, affinity chromatography steps, hydrophobic interaction chromatography steps, dye chromatography steps, and size exclusion chromatography steps.

In order to minimize the theoretical risk of virus contaminations, additional steps may be included in the process that allows effective inactivation or elimination of viruses. Such steps for example comprise heat treatment in the liquid or solid state, treatment with solvents and/or detergents, radiation in the visible or UV spectrum, gamma-radiation or nanofiltration.

The Factor VIII proteins as described in this invention can be formulated into pharmaceutical preparations for therapeutic use. The purified protein may be dissolved in conventional physiologically compatible aqueous buffer solutions to which there may be added, optionally, pharmaceutical excipients to provide pharmaceutical preparations.

Such pharmaceutical carriers and excipients as well as suitable pharmaceutical formulations are well known in the art (see for example "Pharmaceutical Formulation Development of Peptides and Proteins", Frokjaer et al., Taylor & Francis (2000) or "Handbook of Pharmaceutical Excipients", 3rd edition, Kibbe et al., Pharmaceutical Press (2000)). In particular, the pharmaceutical composition comprising the polypeptide variant of the invention may be formulated in lyophilized or stable liquid form. The polypeptide variant may be lyophilized by a variety of procedures known in the art. Lyophilized formulations are reconstituted prior to use by the addition of one or more pharmaceutically acceptable diluents such as sterile water for injection or sterile physiological saline solution.

Formulations of the composition are delivered to the individual by any pharmaceutically suitable means of administration. Various delivery systems are known and can be used to administer the composition by any convenient route. Preferentially, the compositions of the invention are administered systemically. For systemic use, proteins of the invention are formulated for parenteral (e.g. intravenous, subcutaneous, intramuscular, intraperitoneal, intracerebral, intrapulmonar, intranasal or transdermal) or enteral (e.g., oral, vaginal or rectal) delivery according to conventional methods. The most preferential routes of administration are intravenous and subcutaneous administration. The formulations can be administered continuously by infusion or by bolus injection. Some formulations encompass slow release systems.

The pharmaceutical compositions can be used to treat medical conditions preferably hemophila A, wherein the hemophilia A can be congenital hemophilia A or acquired hemophilia A. Also encompassed is the treatment of Hemophilia A with inhibitors encompassing the high dose treatment in order to induction tolerance to inhibitory antibodies also called ITT for immune tolerance therapy.

The pharmaceutical composition of the invention may be administered alone or in conjunction with other therapeutic agents. These agents may be incorporated as part of the same pharmaceutical. One example of such an agent is von Willebrand factor.

### Manufacture of pharmaceutical FVIII preparations free of non-associated FVIII-LCs

For the manufacture of pharmaceutical Factor VIII preparations free of non-associated FVIII-LCs tc-FVIII is applied onto a SE-HPLC or SEC column and eluted using an eluent suitable for maintaining native conditions at a physiological pH under isocratic conditions. SEC resins suitable for preparative SEC at different manufacturing scales are e.g. Superdex 200^{®}, Sephacryl S 200^{®} (GE Healthcare) and others known to the expert in the field. Non-associated FVIII-LCs elute later than the associated tc-FVIII due to a lower molecular weight of the protein. Therefore, early eluting fractions are devoid of non-associated FVIII-LCs. The eluting peak is fractionated and the collected fractions are analyzed regarding non-associated FVIII-LCs by analytical SE-HPLC using an eluent at physiological pH under isocratic conditions. Fractions free of non-associated FVIII-LCs are identified by the lack of non-associated FVIII-LCs. To obtain pharmaceutical FVIII preparations essentially free of non-associated FVIII-LCs, the fractions shown to be free of non-associated FVIII-LCs may be pooled, buffer exchanged against formulation buffer, activity adjusted, sterile filtered and freeze-dried.

### Manufacture of pharmaceutical FVIII preparations free of non-associated FVIII-HCs

The detection of non-associated FVIII-HCs in tc-FVIII preparations is carried out using an analytical SE-HPLC column suitable for the separation of non-associated FVIII-HCs from non-associated FVIII-LCs. To obtain information on the retention time range for non-associated FVIII-HCs, spike recovery experiments using tc-FVIII preparations which are supplemented with non-associated FVIII-HCs are carried out. To obtain non-associated FVIII-HCs, a tc-FVIII preparation is subjected to SE-HPLC or SEC using a mobile phase supplemented with EDTA for the disruption of the metal-dependent association of the FVIII-HCs and FVIII-LCs. The released FVIII-HCs elute as a peak prior to the FVIII-LCs due to the higher molecular weight. The peak is fractionated and the collected fractions are buffer exchanged against a physiological buffer lacking EDTA for removal of the chelating agent. The collected fractions are analyzed by analytical SE-HPLC for the presence of non-associated FVIII-HCs. Selected fractions may also be analyzed by RP-HPLC or Edman-sequencing for identity confirmation purposes. Fractions solely containing non-associated FVIII-HCs can be employed for spike recovery experiments.

To obtain pharmaceutical FVIII preparations essentially free of non-associated FVIII-HCs, the tc-FVIII preparation is applied onto a SE-HPLC or SEC column and eluted using an eluent at physiological pH under isocratic conditions. The analyte is detected either with a UV detector set at a wavelength of 280 nm or by using a fluorescence detector with the excitation wavelength set at 280 nm and detection of the emission at 340 nm. The eluting peak is fractionated and the collected fractions are analyzed towards non-associated FVIII-HCs by analytical SE-HPLC using an eluent at physiological pH under isocratic conditions and a column suitable for the separation of non-associated FVIII-HCs from FVIII-LCs.

Non-associated FVIII-HCs elute after tc-FVIII and prior to non-associated FVIII-LCs. For identity confirmation purposes selected fractions can be supplemented with non-associated FVIII-HCs. Selected fractions may also be analyzed by RP-HPLC or Edman-sequencing for identity confirmation purposes. To obtain a pharmaceutical Factor VIII essentially free of non-associated FVIII-HCs the SE-HPLC or SEC fractions, respectively, lacking non-associated FVIII-HCs may be pooled, buffer exchanged against formulation buffer, activity adjusted, sterile filtered and freeze-dried.

Alternatively for analysis as well as for preparation, the collected fractions are applied individually onto an analytical immunoaffinity chromatography (IAC) column consisting of an immobilized antibody or antibody related molecule directed against the FVIII-LCs. Heterodimeric tc-FVIII molecules composed of associated FVIII-HCs and FVIII-LCs are immobilized on the column via binding of the FVIII-LCs to the affinity ligand. Potentially non-associated FVIII-HCs are not retained on the column and elute in the flowthrough fraction. The flowthrough fractions are subsequently analyzed by means of RP-HPLC in combination with Edman-sequencing for identity confirmation purposes and for the detection of non-associated FVIII-HCs. To obtain a pharmaceutical Factor VIII preparation free of non-associated FVIII-HCs the SE-HPLC or SEC fractions, respectively, lacking non-associated FVIII-HCs (as shown by IA-chromatography in combination with RP-HPLC and/or Edman-sequencing) may be pooled, buffer exchanged against formulation buffer, activity adjusted, sterile filtered and freeze-dried.

### Size-exclusion chromatography (SEC)

Size-exclusion chromatography (SEC) is a method that can be applied for the purification and/or analysis of proteins under native conditions. Depending on the particle size of the separation resin used, the SEC is performed as a SE-HPLC where smaller resin particles and higher pressures are applied or as a SEC where larger resin particles and lower or medium pressures are applied. Suitable SEC resins for preparative purposes are e.g. Superdex 200^{®}, Sepahycryl S 200^{®} (GE Healthcare) or other known to the expert in the field with appropriate MW range. The proteins are separated according to their size, which in turn correlates with the respective molecular weight. The stationary phase used for SE-HPLC or SEC, respectively, is generally a porous solid support with a defined pore size distribution. The pore size defines which molecules can permeate the particles of the stationary phase and thus are retained on the column. Large molecules that are not capable of diffusing into the pores elute earlier, whereas smaller molecules are retained on the column and elute in a higher retention time range. Typical column packing materials are cross-linked dextran-, polyacrylamide- and agarose-supports or porous silica beads. The eluent, also referred to as the mobile phase, is typically a buffer stabilizing the protein in solution and generally does not have a direct effect on analyte separation. The SEC-eluent is typically composed of salts (e.g. NaCl), buffering agents (e.g. histidine, Tris, HEPES, imidazole), buffer additives for the stabilization of the protein (e.g. Polysorbate 80, PEG) and in some cases eluent additives (e.g. 2-propanol). For protein analysis the buffer is commonly adjusted to a physiological pH-value and the method is carried out under isocratic conditions. Since the protein does not directly interact with the stationary phase and is not subjected to denaturing conditions due to the use of a physiological buffer and moderate temperatures size exclusion chromatography is considered a native method. Under these conditions, non-covalent protein-protein interactions/associations and protein complexes remain unaffected in contrast to denaturing methods.

### Immunoaffinity chromatography

Immunoaffinity chromatography represents a sub-category of affinity chromatography which relies on the highly specific interaction of an antibody or antibody related molecule with the target compound. This type of chromatography can be carried out for either preparative or analysis purposes of a target compound under native conditions. Molecules which lack affinity towards the immobilized antibody or antibody related molecule are not retained on the column, making IAC a highly specific method for the analysis or purification of the target compound. The target compound that is immobilized on the column can subsequently be eluted by using appropriate elution buffers.

### Examples

### Example 1: Chromogenic Factor VIII activity assay

Factor VIII activity was measured by its highly specific physiological function in the activation of FX as cofactor of FIXa (according to Ph. Eur.2.7.4.). In the presence of calcium and phospholipids, Factor X is activated by Factor IXa to Factor Xa. This reaction is stimulated by Factor Villa as cofactor. Factor Villa is formed by low amounts of thrombin in the reaction mixture from Factor VIII in the sample to be measured. Activated Factor X releases the chromophore pNA from the chromogenic substrate S-2765. The release of pNA, measured at 405 nm, is therefore proportional to the Factor VIII activity of the sample. The activity of Coagulation Factor VIII was quantified by direct comparison to a standard preparation with a known activity of Factor VIII calibrated against the current WHO reference preparation. The assay was carried out on a Behring Coagulation System (BCS, Siemens) operated according to the manufacturer's instructions and established protocols.

### Example 2: N-terminal sequencing (Edman-sequencing)

The N-terminal sequence of the products was determined by successive chemical cleavage and derivatization of the N-terminal amino acids. The derivatized amino acids were subsequently separated by RPC followed by UV-based identification and quantification. As a result, the sequence of the twelve N-terminal amino acids present in the analyzed product was reported. Edman-sequencing was carried out on a Protein Sequencer Procise 492^{®} (ABI) according to the protocol provided by the manufacturer. The identity of the released and derivatized amino acids was confirmed employing a commercially available PTH-derivatized amino acid standard mixture.

### Example 3: Analytical SE-HPLC assays and analysis of different commercially available FVIII products

Analytical Size-Exclusion-High Performance Liquid Chromatography (SE-HPLC) analysis of different commercially available FVIII products was carried out using an analytical SE-HPLC column (COSMOSIL 5Diol-300-II^{®}, 600 mm x 7.5 mm, 300 Å pore size, 5 µm particle size, Nacalai Tesque) in combination with a guard column (SecurityGuard Guard Cartridge System^{®}, GFC 3000, 4 mm x 3 ID mm, Phenomenex) and a mobile phase (300 mM NaCl, 20 mM HEPES, 10 mM CaCl2, 0.005% Polysorbate 80, 10 % isopropanol) at neutral pH (7.0) under isocratic conditions on a System Gold instrument (Beckman Coulter). The instrument was operated using a flow rate of 500 µL/min and the column was operated at room temperature. The analytes were detected using a fluorescence detector (FLD) set at an excitation wavelength of 280 nm and detection of the emission at 340 nm. Relative quantification of non-associated FVIII-LCs in BDD rFVIII products (ReFacto AF@ (Pfizer) and Novo8^{®} (Novo Nordisk)) was carried out on an Ultimate 3000 HPLC instrument (Dionex Thermo Fisher) using the method parameters described above.

Additionally, an alternative SE-HPLC-column (YARRA SEC-3000^{®}, 300 mm x 7.8 mm, 290Å pore size, 3 µm particle size, Phenomenex) in combination with a guard column (SecurityGuard Guard Cartridge System^{®}, GFC 3000, 4 mm x 3 ID mm, Phenomenex) and a mobile phase (300 mM NaCl, 20 mM HEPES, 10 mM CaCl2, 0.005% Polysorbate 80) at neutral pH (7.0) was utilized under isocratic conditions on a System Gold HPLC instrument (Beckman Coulter). The instrument was operated using a flow rate of 300 µL/min and the column was operated at room temperature. The analytes were detected using a fluorescence detector (FLD) set at an excitation wavelength of 280 nm and detection of the emission at 340 nm.

### Example 4: Preperative isolation of rFVIII free of FVIII-LCs which are non-associated with FVIII-HCs (full-length rFVIII (Advate^{®}))

Full-length rFVIII (Advate^{®}, 350-400 IU on column in 100 µL) was applied onto a SE-HPLC column (YARRA SEC-3000^{®}, 300 mm x 7.8 mm, 290 Å pore size, 3 µm particle size, Phenomenex) in combination with a guard column (SecurityGuard Guard Cartridge System^{®}, Security Guard Cartridge, GFC 3000, 4 mm x 3 ID mm, Phenomenex) and eluted using a mobile phase (300 mM NaCl, 20 mM HEPES, 10 mM CaCl₂, 0.005% Polysorbate 80) at neutral pH (7.0) under isocratic conditions on a System Gold HPLC instrument (Beckman Coulter). The instrument was operated using a flow rate of 300 µL/min and the column was operated at room temperature. The analytes were detected using a fluorescence detector (FLD) set at an excitation wavelength of 280 nm and detection of the emission at 340 nm. A late eluting fraction (30.5 - 34 min) was collected (Figure 2). This fractionation approach was carried out six times and the collected fractions were pooled. The pool was subjected to Edman sequencing for identity confirmation purposes. Two protein species could be identified based on the N-terminal sequence by Edman sequencing, both representing FVIII-LCs (Table 1). The fraction may also be analyzed by RP-HPLC for identity confirmation purposes.

**Table 1: Results of the N-terminal sequencing of the late eluting fraction.**

| Main sequence (determined) | E | I | T | R | T | T | L | Q | S | D | Q | E |
|---|---|---|---|---|---|---|---|---|---|---|---|---|
| FVIII sequence | E | I | T | R | T | T | L | Q | S | D | Q | E |
| Additional sequence (determined) | D | X¹ | E | E | I | D | X¹ | X¹ | D | T | I | X¹ |
| FVIII sequence | D | Q | E | E | I | D | Y | D | D | T | I | S |

| | | | | | | | | | | | | |
|---|---|---|---|---|---|---|---|---|---|---|---|---|
| ¹unambigous determination of amino acid residue via pmol-increase not feasible | | | | | | | | | | | | |

After confirming that the late eluting fraction contains non-associated FVIII-LCs a relative quantification of the FVIII-LCs separated in SE-HPLC-analysis was carried out. The results are summarized in Table 2. Advate^{®} comprises an amount of approx. 5% non-associated FVIII-LCs based on relative quantification of the non-fractionated starting material.

**Table 2: Results obtained for the relative quantification of non-associated FVIII-LCs observed during preparative SE-HPLC of Advate^{®}.**

| injection | amount of non-associated FVIII-LCs (%) |
|---|---|
| 1 | 5.3 |
| 2 | 5.0 |
| 3 | 5.1 |
| 4 | 5.6 |
| mean value (n = 4) | 5.3 |

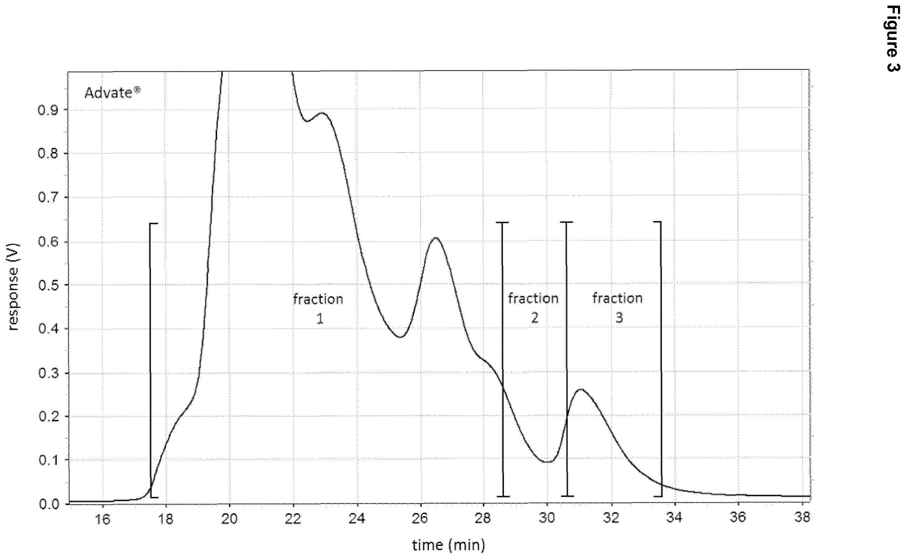

In an additional approach Advate^{®} was fractionated following the established protocol and three fractions of the elution peak were collected (Figure 3 and Table 3). The fractionation was repeated four times and identical fractions 1, 2 and 3 were pooled.

**Table 3: Representative retention time ranges of the respective fractions.**

| fraction (designation) | retention time range (min) |
|---|---|
| 1 | 17.5 - 28.6 |
| 2 | 28.6 - 30.6 |
| 3 | 30.6 - 33.6 |

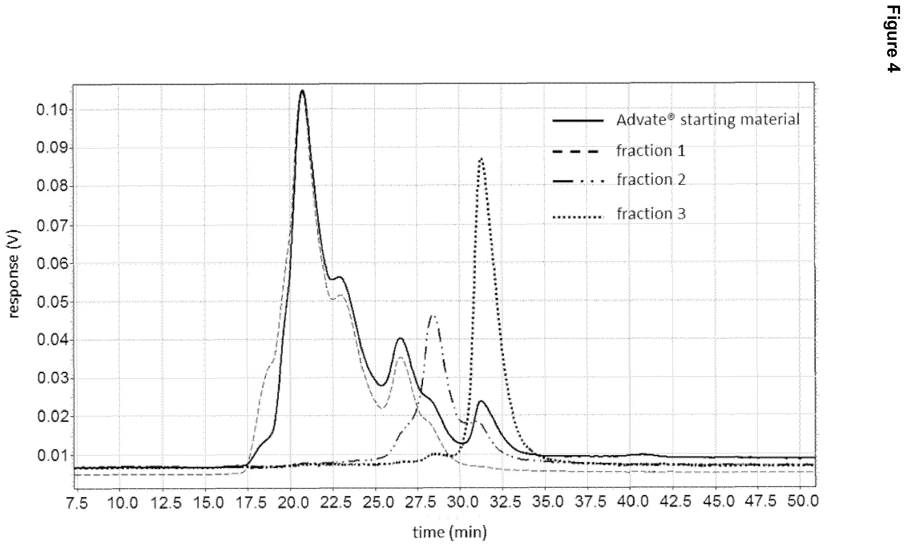

These three fractions were analyzed for the presence of non-associated FVIII-LCs, i.e. being not associated with their respective counterpart via a metal dependent association by analytical SE-HPLC (Size Exclusion HPLC) using an analytical SE-HPLC column (YARRA SEC-3000^{®}, 300 mm x 7.8 mm, 290 Å pore size, 3 µm particle size, Phenomenex) in combination with a guard column (SecurityGuard Guard Cartridge System^{®}, GFC 3000, 4 mm x 3 ID mm, Phenomenex) and using a mobile phase (300 mM NaCl, 20 mM HEPES, 10 mM CaCl₂, 0.005% Polysorbate 80) at neutral pH (7.0) under isocratic conditions on a System Gold HPLC instrument (Beckman Coulter) (see Figure 4). The instrument was operated using a flow rate of 300 µL/min and the column was operated at room temperature. Selected fractions may also be analyzed by RP-HPLC or Edman sequencing for identity confirmation purposes.

Combined fractions 1 were shown by analytical SE-HPLC to be essentially free of non-associated FVIII-LCs, whereas combined fractions 3 contained predominantly non-associated FVIII-LCs.

In addition, no newly formed, non-associated FVIII-LCs can be observed during SE-HPLC-analysis of combined fractions 1, thus confirming the stability of the heterodimeric FVIII-LC/HC complex in which the FVIII-LCs are associated with their respective FVIII-HC counterparts.

The FVIII-LCs in fraction 3 were shown to be non-associated with FVIII- due to non-denaturing conditions applied during SEC (native SEC).

To obtain a pharmaceutical Factor VIII preparation essentially free of non-associated FVIII-LCs the pooled rFVIII fractions 1 may then be buffer exchanged against formulation buffer, activity adjusted, sterile filtered and freeze-dried.

### Example 5: Preparative isolation of rFVIII free of non-associated FVIII-LCs (BDD rFVIII (ReFacto AF^{®}))

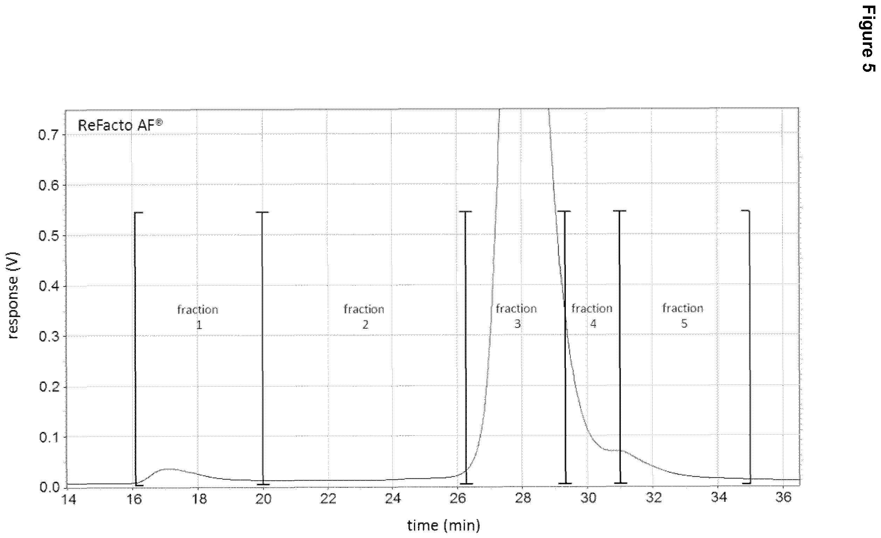

BDD rFVIII (ReFacto AF^{®}, 525 IU on column in 70 µL) was applied onto a SE-HPLC column (YARRA SEC-3000^{®}, 300 mm x 7.8 mm, 290 Å pore size, 3 µm particle size, Phenomenex) in combination with a guard column (SecurityGuard Guard Cartridge System^{®}, GFC 3000, 4 mm x 3 ID mm, Phenomenex) and eluted using a mobile phase (300 mM NaCl, 20 mM HEPES, 10 mM CaCl₂, 0.005% Polysorbate 80) at neutral pH (7.0) under isocratic conditions on a System Gold HPLC instrument (Beckman Coulter). The instrument was operated using a flow rate of 300 µL/min and the column was operated at room temperature. The analytes were detected using a fluorescence detector (FLD) set at an excitation wavelength of 280 nm and detection of the emission at 340 nm. Five fractions of the elution peak were collected (Figure 5 and Table 4).

**Table 4: Retention time ranges of the respective fractions.**

| fraction | retention time range (min) |
|---|---|
| 1 | 16.1 -20.0 |
| 2 | 20.0 - 26.3 |
| 3 | 26.3 - 29.3 |
| 4 | 29.3 - 31.0 |
| 5 | 31.0 - 35.0 |

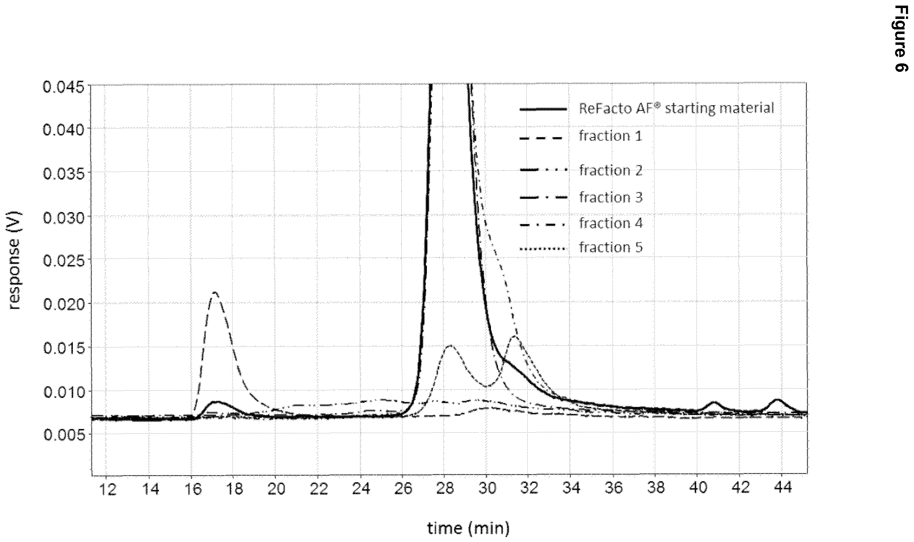

The fractionation was repeated six times and identical fractions 1 to 5 were pooled. The collected fractions were analyzed for the presence of non-associated FVIII-LCs by analytical SE-HPLC using an analytical SE-HPLC column (YARRA SEC-3000^{®}, 300 mm x 7.8 mm, 290 Å pore size, particle size 3µm, Phenomenex) in combination with a guard column (SecurityGuard Guard Cartridge System^{®}, GFC 3000, 4 mm x 3 ID mm, Phenomenex) and eluted using a mobile phase (300 mM NaCl, 20 mM HEPES, 10 mM CaCl₂, 0.005% Polysorbate 80) at neutral pH (7.0) under isocratic conditions on a System Gold HPLC instrument (Beckman Coulter) (see Figure 6). The instrument was operated using a flow rate of 300 µL/min and the column was operated at room temperature. Selected fractions may also be analyzed by RP-HPLC or Edman-sequencing for identity confirmation purposes.

Combined fractions 3 contained a stable heterodimer of tc-FVIII and were shown by analytical SE-HPLC to be essentially free of FVIII-LCs which is non-associated with FVIII-HCs. No newly formed, non-associated FVIII-LCs can be observed during SE-HPLC-analysis of combined fractions 3, thus confirming the stability of the heterodimeric FVIII-LC/HC complex in which the FVIII-LCs are associated with their respective FVIII-HC counterparts.

In contrast, combined fractions 5 comprise FVIII-LCs which are not associated with FVIII-HCs (at appr. 31.5 min) and another peak at approximately 30.5 min which contains tc-FVIII. The FVIII-LCs in Fraction 5 at 31.5 min were non-associated with FVIII-HCs due to the non-denaturing conditions applied during SE-HPLC (native SE-HPLC). In addition, a relative quantification of non-associated FVIII-LCs observed during SEC analysis of ReFacto AF^{®} was carried out on an Ultimate 3000 System (see Example 3) using non-fractionated material. The results are summarized in Table 5.

To obtain a pharmaceutical Factor VIII preparation essentially free of non-associated FVIII-LCs the pooled rFVIII fractions 3 may be buffer exchanged against formulation buffer, activity adjusted, sterile filtered and freeze-dried.

**Table 5: Results obtained for the relative quantification of non-associated FVIII-LCs observed during SE-HPLC analysis of ReFacto AF^{®}.**

| injection | amount of non-associated FVIII-LCs (%) |
|---|---|
| 1 | 0.9 |
| 2 | 1.0 |
| 3 | 1.0 |
| 4 | 0.8 |
| mean value (n = 4) | 0.9 |

### Example 6: Preparative isolation of rFVIII free of non-associated FVIII-LCs using a Superdex^{®} SEC column

B-Domain deleted (BDD) rFVIII (ReFacto AF^{®}, 1200 IU on column in 400 µL) was applied onto an Superdex SEC column (Superdex^{®} 200 10/300 GL, 300 mm x 10 mm, 24 mL bed volume, average particle size 13 µm, GE Healthcare) and eluted using a mobile phase (300 mM NaCl, 20 mM HEPES, 10 mM CaCl₂, 0.005% Polysorbate 80) at neutral pH (7.0) under isocratic conditions on an ICS-3000 chromatography system (Dionex Thermo Fisher). The instrument was operated using a flow rate of 300 µL/min and the column was operated at room temperature. The analytes were detected using a UV detector (VWD) set at 280 nm. BDD rFVIII was applied in a volume of approximately 2% of the column volume (400 µL). Fractions of the eluting peak were collected and pooled according to the scheme outlined in Table 6.

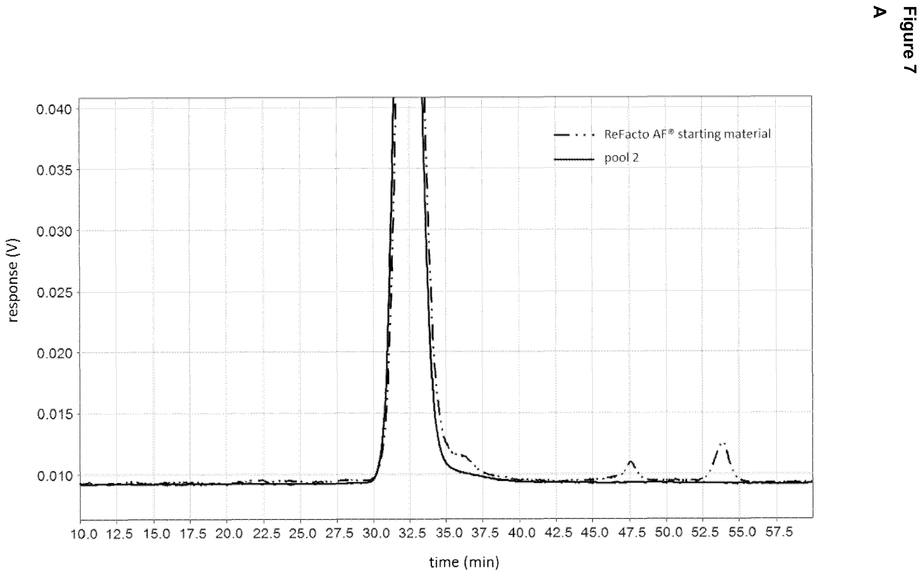

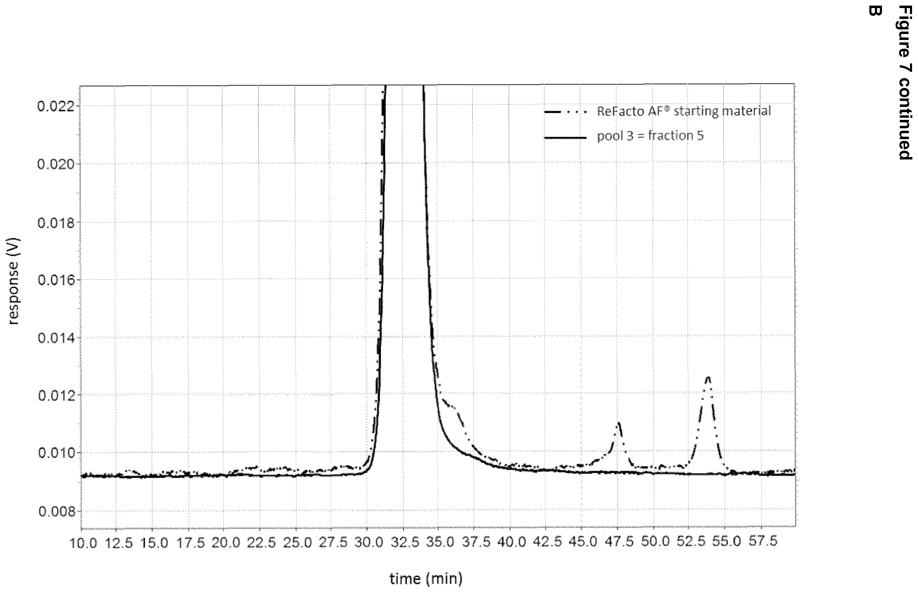

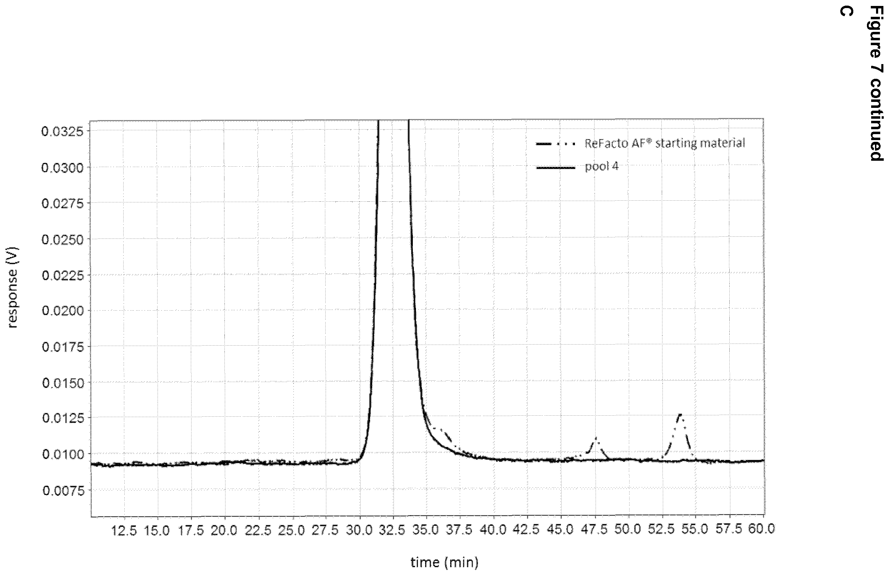

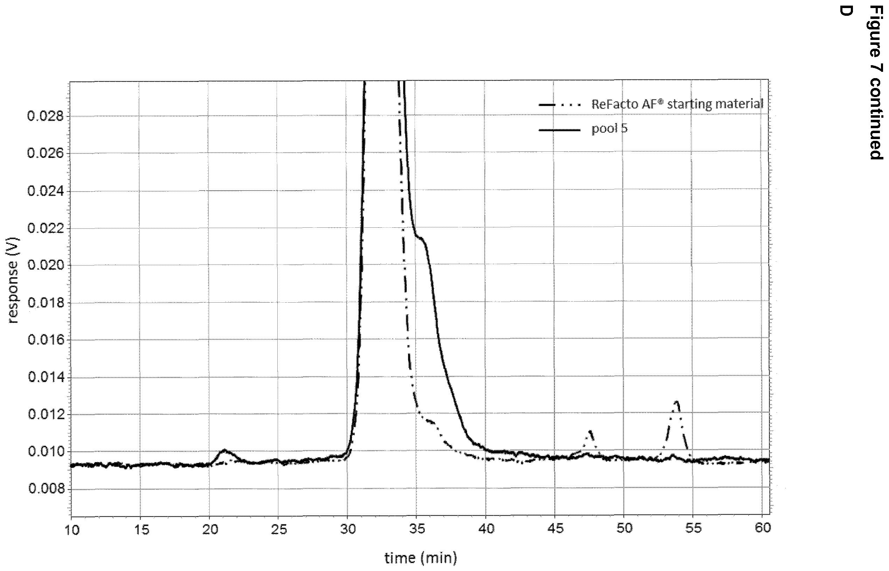

The pooled fractions were analyzed for chromogenic substrate FVIII activity using a BCS according to the procedure outlined in Example 1. The results are summarized in Table 7. Pools 2-4 were found to comprise the majority of FVIII activity with about 86.1% of the FVIII activity applied to the column or 90.8% of the FVIII:CS activity recovered in all fractions. These relevant pools/fractions were also analyzed by analytical SE-HPLC for the presence of non-associated FVIII-LCs. This SE-HPLC-analysis was carried out using an analytical SE-HPLC column (COSMOSIL 5Diol-300-II^{®}, 600 mm x 7.5 mm, 300 Å pore size, 5 µm particle size, Nacalai Tesque) in combination with a guard column (SecurityGuard Guard Cartridge System^{®}, GFC 3000, 4 mm x 3 ID mm, Phenomenex) and a mobile phase (300 mM NaCl, 20 mM HEPES, 10 mM CaCl₂, 0.005% Polysorbate 80, 10% isopropanol) at neutral pH (7.0) under isocratic conditions on a System Gold HPLC instrument (Beckman Coulter). The instrument was operated using a flow rate of 500 µL/min and the column was operated at room temperature. The analytes were detected using a fluorescence detector (FLD) set at an excitation wavelength of 280 nm and detection of the emission at 340 nm (Figures 7a-d). Analysis of the pooled fractions 2-4 by analytical SE-HPLC demonstrated that the FVIII preparations in pools 2-4 were essentially free of non-associated FVIII-LCs, whereas pool 5 was found to contain non-associated FVIII-LCs. In addition, no newly formed, non-associated FVIII-LCs can be observed during SE-HPLC-analysis of pools 2-4, thus confirming the stability of the heterodimeric FVIII-LC/HC complex in which the FVIII-LCs are associated with their respective FVIII-HC counterparts.

Selected fractions may also be analyzed by RP-HPLC or Edman sequencing for identity confirmation purposes.

To obtain a pharmaceutical Factor FVIII preparation essentially free of FVIII-LCs the pooled rFVIII fractions of pool 2 to pool 4 may be buffer exchanged against formulation buffer, activity adjusted, sterile filtered and freeze-dried.

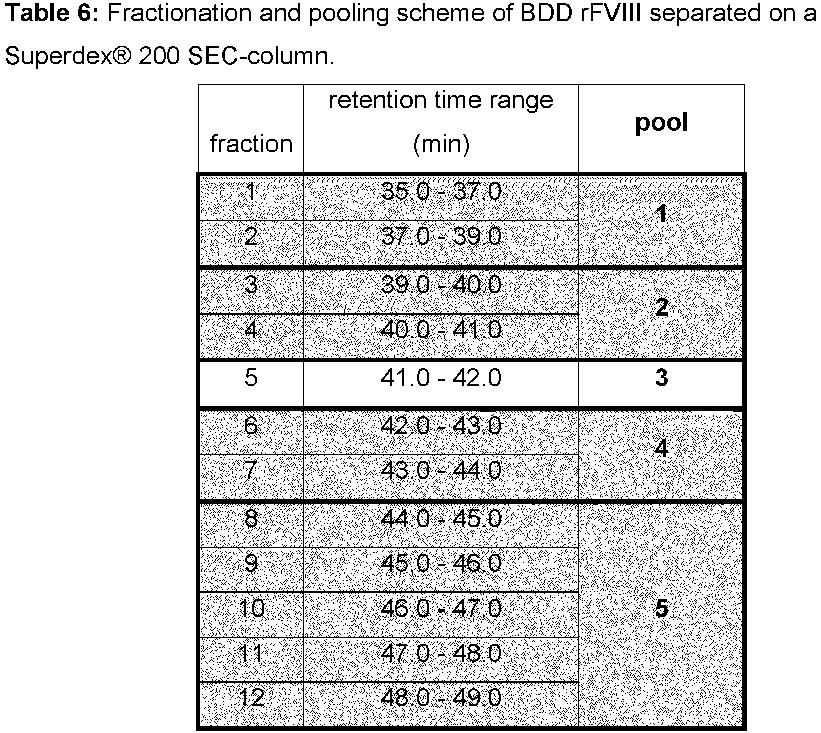

**Table 7: Overview of the chromogenic substrate FVIII activity determined for the collected fractions/pools.**

| | target based on nominal activity (lU/mL) | actually determined (lU/mL) | ratio actual/ target activity | volume (pool/fraction) | FVIII activity per pool or faction (lU/pool or fraction) | |
|---|---|---|---|---|---|---|
| starting material | *3000* | 2395 | 0.798 | 400 µL | 958 | |
| pool 1 | 137 | 114 | 0.832 | approx. 250 µL | 29 | |
| pool 2 | 651 | 620 | 0.952 | 600 µL | 372 | |
| pool 3/ fraction 5 | 811 | 822 | 1.014 | 300 µL | 247 | |
| pool 4 | 336 | 343 | 1.021 | 600 µL | 206 | |
| pool 5 | 18 | 221 | 12.278 | approx. 250 µL | 55 | |
| sum of all pools (IU) | | | | | 909 | |
| sum of pools 2-4 (IU) | | | | | 825 | |
| | | | | | recovery of pool 2-4 regarding total FVIII:CS activity applied onto column (%) | 86.1 |
| | | | | | recovery of pool 2-4 regarding FVIII:CS activity of all fractions (%) | 90.8 |

### Example 7: Determination of non-associated FVIII-LCs in NovoEight^{®} (Novo Nordisk)

Relative quantification of non-associated FVIII-LCs in NovoEight^{®} (500 IU presentation, Novo Nordisk) was carried out on an Ultimate 3000 HPLC instrument (Dionex Thermo Fisher) using an analytical SE-HPLC column (COSMOSIL 5Diol-300-II^{®}, 600 mm x 7.5 mm, 300 Å pore size, 5 µm particle size, Nacalai Tesque) in combination with a guard column (SecurityGuard Guard Cartridge System^{®}, GFC 3000, 4 mm x 3 ID mm, Phenomenex) and a mobile phase (300 mM NaCl, 20 mM HEPES, 10 mM CaCl₂, 0.005% Polysorbate 80, 10 % isopropanol) at neutral pH (7.0) under isocratic conditions. The instrument was operated using a flow rate of 500 µL/min and the column was operated at room temperature. The analytes were detected using a fluorescence detector (FLD) set at an excitation wavelength of 280 nm and detection of the emission at 340 nm. The amount of non-associated FVIII-LCs was determined to be 1.6-1.7%.

### Example 8: Manufacture of pharmaceutical Factor VIII preparations free of non-associated FVIII-HCs

For the manufacture of pharmaceutical Factor VIII preparations free of non-associated FVIII-HCs tc-FVIII is applied onto a SEC column (e.g. Superdex^{®} 200 10/300 GL, 300 mm x 10 mm, 24 mL bed volume, average particle size 13 µm, GE Healthcare, COSMOSIL 5Diol-300-II^{®}, 600 mm x 7.5 mm, 300 Å pore size, 5 µm particle size, Nacalai Tesque, YARRA SEC-3000^{®}, 300 mm x 7.8 mm, 290 Å pore size, 3 µm particle size, Phenomenex; or other suitable prep-grade SEC or SE-HPLC resins and eluted using an eluent suitable for maintaining native conditions at a physiological pH under isocratic conditions. The analytes are detected either with a UV detector set at a wavelength of 280 nm or by using a fluorescence detector with the excitation wavelength set at 280 nm and detection of the emission at 340 nm. The eluting peak is fractionated and the collected fractions are analyzed regarding non-associated FVIII-HCs.

For this purpose, the collected fractions are applied individually onto an analytical immunoaffinity chromatography (IAC) column consisting of an immobilized antibody or antibody related molecule directed against the FVIII-LCs. Heterodimeric tc-FVIII molecules composed of associated FVIII-HCs and FVIII-LCs are immobilized on the column via binding of the FVIII-LCs to the affinity ligand. Potentially non-associated FVIII-HCs are not retained on the column and elute in the flowthrough fraction. The flowthrough fractions are subsequently analyzed by means of RP-HPLC in combination with Edman-sequencing for identity confirmation purposes and for the detection of non-associated FVIII-HCs. Collected fractions may also be analysed by analytical SE-HPLC for the presence of non-associated FVIII-HCs. To obtain a pharmaceutical Factor VIII preparation free of non-associated FVIII-HCs the SEC fractions lacking non-associated FVIII-HCs (as shown by IA-chromatography in combination with RP-HPLC and/or Edman-sequencing) may be buffer exchanged against formulation buffer, activity adjusted, sterile filtered and freeze-dried.

## Claims

1. Pharmaceutical preparation comprising two-chain Factor VIII (tc-FVIII) wherein less than 0.9% of all FVIII-LCs within said preparation are non-associated with FVIII-HCs wherein non-associated FVIII-LCs mean FVIII-LCs which are not part of a stable heterodimer with FVIII-HCs and which non-associated FVIII-LCs will therefore not elute as part of a heterodimer in a native size-exclusion chromatography but rather as monomers of non-associated FVIII-LCs, obtainable by a size exclusion chromatography (SEC) wherein a tc-FVIII is i) applied onto a SE-HPLC or SEC column and eluted using an eluent suitable for maintaining native conditions at a physiological pH under isocratic conditions, ii) early eluting peaks are fractionated and iii) the collected fractions are analyzed regarding non-associated FVIII-LCs by analytical SE-HPLC using an eluent at physiological pH under isocratic conditions, iv) fractions free of non-associated FVIII-LCs are identified by the lack of non-associated FVIII-LCs and are optionally pooled, buffer exchanged against formulation buffer, activity adjusted, sterile filtered and freeze-dried.

2. Pharmaceutical preparation according to claim 1 wherein the tc-FVIII is a full-length Factor VIII.

3. Pharmaceutical preparation according to claims 1 to 2 wherein the tc-FVIII is a full-length Factor VIII of plasmatic origin.

4. Pharmaceutical preparation according to claims 1 to 2 wherein the tc-FVIII is a full-length Factor VIII of recombinant origin.

5. Pharmaceutical preparation according to claims 3 to 4 wherein the molecular weight of the non-associated FVIII-LCs is about 70 to 125 kDa.

6. Pharmaceutical preparation according to claim 1 wherein the tc-FVIII is a B-domain truncated Factor VIII.

7. Pharmaceutical preparation according to claim 6 wherein the molecular weight of the non-associated FVIII-LCs is about 70 to 125 kDa.

8. Pharmaceutical preparation according to claims 1 to 7 wherein the pharmaceutical preparation is less immunogenic as compared to a pharmaceutical preparation comprising tc-FVIII wherein 0.9% or more of all FVIII-LCs within said preparation are non-associated with FVIII-HCs wherein non-associated FVIII-LCs mean FVIII-LCs which are not part of a stable heterodimer with FVIII-HCs and which non-associated FVIII-LCs will therefore not elute as part of a heterodimer in a native size-exclusion chromatography but rather as monomers of non-associated FVIII-LCs.

9. Pharmaceutical preparation according to claims 1 to 8 wherein the Factor VIII is human Factor VIII.

10. Pharmaceutical preparation according to claims 1 to 9 for use in medicine.

11. Pharmaceutical preparation according to claim 1 to 10 for use in the treatment of hemophilia A.

## Patentansprüche

1. Pharmazeutisches Präparat, umfassend zweikettigen Faktor VIII (two-chain Factor VIII, tc-FVIII), wobei weniger als 0,9% aller FVIII-LC in dem Präparat nicht mit FVIII-HC assoziiert sind, wobei unter nicht assoziierten FVIII-LC FVIII-LC verstanden werden, die nicht Teil eines stabilen Heterodimers mit FVIII-HC sind, und wobei nicht assoziierte FVIII-LC daher bei einer nativen Größenausschlusschromatographie nicht als Teil eines Heterodimers, sondern vielmehr als Monomere von nicht assoziierten FVIII-LC eluieren, die über eine Größenausschlusschromatographie (Size Exclusion Chromatography, SEC) erhältlich sind, wobei ein tc-FVIII i) auf eine SE-HPLC- oder SEC-Säule aufgetragen und unter Verwendung eines Elutionsmittels mit Eignung zum Aufrechterhalten nativer Bedingungen bei einem physiologischen pH unter isokratischen Bedingungen eluiert wird, ii) früh eluierende Peaks fraktioniert werden und iii) die gesammelten Fraktionen bezüglich nicht assoziierter FVIII-LC mittels analytischer SE-HPLC unter Verwendung eines Elutionsmittels bei physiologischem pH unter isokratischen Bedingungen analysiert werden, iv) von nicht assoziierten FVIII-LC freie Fraktionen anhand des Fehlens nicht assoziierter FVIII-LC identifiziert und gegebenenfalls vereinigt, einem Pufferaustausch gegen Formulierungspuffer unterzogen, aktivitätseingestellt, sterilfiltriert und gefriergetrocknet werden.

2. Pharmazeutisches Präparat nach Anspruch 1, wobei es sich bei dem tc-FVIII um einen Faktor VIII in voller Länge handelt.

3. Pharmazeutisches Präparat nach Anspruch 1 bis 2, wobei es sich bei dem tc-FVIII um einen aus Plasma stammenden Faktor VIII in voller Länge handelt.

4. Pharmazeutisches Präparat nach Anspruch 1 bis 2, wobei es sich bei dem tc-FVIII um einen Faktor VIII in voller Länge rekombinanten Ursprungs handelt.

5. Pharmazeutisches Präparat nach Anspruch 3 bis 4, wobei das Molekulargewicht der nicht assoziierten FVIII-LC etwa 70 bis 125 kDa beträgt.

6. Pharmazeutisches Präparat nach Anspruch 1, wobei es sich bei dem tc-FVIII um einen Faktor VIII mit B-Domäne-Verkürzung handelt.

7. Pharmazeutisches Präparat nach Anspruch 6, wobei das Molekulargewicht der nicht assoziierten FVIII-LC etwa 70 bis 125 kDa beträgt.

8. Pharmazeutisches Präparat nach Anspruch 1 bis 7, wobei das pharmazeutische Präparat weniger immunogen im Vergleich zu einem pharmazeutischen Präparat ist, das tc-FVIII umfasst, wobei 0,9% oder mehr aller FVIII-LC in dem Präparat nicht mit FVIII-HC assoziiert sind, wobei unter nicht assoziierten FVIII-LC FVIII-LC verstanden werden, die nicht Teil eines stabilen Heterodimers mit FVIII-HC sind, und wobei nicht assoziierte FVIII-LC daher bei einer nativen Größenausschlusschromatographie nicht als Teil eines Heterodimers, sondern vielmehr als Monomere von nicht assoziierten FVIII-LC eluieren.

9. Pharmazeutisches Präparat nach Anspruch 1 bis 8, wobei es sich bei dem Faktor VIII um menschlichen Faktor VIII handelt.

10. Pharmazeutisches Präparat nach Anspruch 1 bis 9 zur medizinischen Verwendung.

11. Pharmazeutisches Präparat nach Anspruch 1 bis 10 zur Verwendung bei der Behandlung von Hämophilie A.

## Revendications

1. Préparation pharmaceutique comprenant un Facteur VIII bicaténaire (tc-FVIII), moins de 0,9 % de tous les FVIII-LC dans ladite préparation n'étant pas associés à des FVIII-HC, des FVIII-LC non associés signifiant des FVIII-LC qui ne font pas partie d'un hétérodimère stable avec des FVIII-HC et lesquels FVIII-LC non associés n'élueront par conséquent pas comme partie d'un hétérodimère dans une chromatographie d'exclusion stérique native mais plutôt comme monomères de FVIII-LC non associés, pouvant être obtenus par une chromatographie d'exclusion stérique (SEC) dans laquelle un tc-FVIII est i) appliqué sur une colonne de SE-HPLC ou SEC et élué en utilisant un éluant approprié pour le maintien de conditions natives à un pH physiologique dans des conditions isocratiques, ii) des pics éluant tôt étant fractionnés et iii) les fractions collectées étant analysées en ce qui concerne des FVIII-LC non associés par SE-HPLC analytique en utilisant un éluant à un pH physiologique dans des conditions isocratiques, iv) des fractions exemptes de FVIII-LC non associés étant identifiées par le manque de FVIII-LC non associés et étant éventuellement regroupées, soumises à un échange de tampon contre un tampon de formulation, ajustées en activité, filtrées stérilement et lyophilisées.

2. Préparation pharmaceutique selon la revendication 1, le tc-FVIII étant un Facteur VIII de pleine longueur.

3. Préparation pharmaceutique selon les revendications 1 à 2, le tc-FVIII étant un Facteur VIII de pleine longueur d'origine plasmatique.

4. Préparation pharmaceutique selon les revendications 1 à 2, le tc-FVIII étant un Facteur VIII de pleine longueur d'origine recombinante.

5. Préparation pharmaceutique selon les revendications 3 à 4, le poids moléculaire des FVIII-LC non associés étant d'environ 70 à 125 kDa.

6. Préparation pharmaceutique selon la revendication 1, le tc-FVIII étant un Facteur VIII tronqué de domaine B.

7. Préparation pharmaceutique selon la revendication 6, le poids moléculaire des FVIII-LC non associés étant d'environ 70 à 125 kDa.

8. Préparation pharmaceutique selon les revendications 1 à 7, la préparation pharmaceutique étant moins immunogène par comparaison avec une préparation pharmaceutique comprenant un tc-FVIII, 0,9 % ou plus de tous les FVIII-LC dans ladite préparation n'étant pas associés à des FVIII-HC, des FVIII-LC non associés signifiant des FVIII-LC qui ne font pas partie d'un hétérodimère stable avec des FVIII-HC et lesquels FVIII-LC non associés n'élueront par conséquent pas comme partie d'un hétérodimère dans une chromatographie d'exclusion stérique native mais plutôt comme monomères de FVIII-LC non associés.

9. Préparation pharmaceutique selon les revendications 1 à 8, le Facteur VIII étant un Facteur VIII humain.

10. Préparation pharmaceutique selon les revendications 1 à 9 pour une utilisation en médecine.

11. Préparation pharmaceutique selon les revendications 1 à 10 pour une utilisation dans le traitement de l'hémophilie A.
